# EUROPEAN PATENT SPECIFICATION

(11) **EP 1 107 737 B1**
(45) Date of publication and mention of the grant of the patent: **28.04.2004**
(21) Application number: 99943130.7
(22) Date of filing: 03.09.1999
(51) Int. Cl.: A61K 9/16, A61M 5/30

(54) **MICROPARTICLES FOR USE IN NEEDLELESS INJECTION**
MICROPARTIKELN ZUR VERWENDUNG IN NADELLOSEN INJEKTION
MICROPARTICULES POUR INJECTION SANS AIGUILLE

(30) Priority: 03.09.1998 GB 9819272
(43) Date of publication of application: 20.06.2001
(60) Divisional application: 04075082.0
(73) Proprietor: Quadrant Drug Delivery Limited, Nottingham, NG11 6JS (GB)
(72) Inventor: OSBORNE, Nicholas, David, Nottingham NG4 2DJ (GB)
(74) Representative: Stevens, Ian Edward
(86) International application number: PCT/GB1999/002930
(87) International publication number: WO 2000/013668

(56) References cited:
- WO-A-91/19485
- WO-A-95/34291
- WO-A-96/03979
- WO-A-97/48485
- JOVER, ISABEL ET AL: "Evaluation, by a Statistically Designed Experiment, of an Experimental Grade of Microcrystalline Cellulose, Avicel 955, as a Technology To Aid the Production of Pellets with High Drug Loading" J. PHARM. SCI. (1996), 85(7), 700-705 , XP002130714
- ZOLFAGHARI, MOHAMMAD ESMAIL ET AL: "Effect of particle shape of acetylsalicylic acid powders on gastric damages in rats" J. PHARM. BELG. (1997), 52(1), 1-6 , XP002130715

## Description

### Field of the Invention

This invention relates to microparticles, methods for their formation and their therapeutic use, especially for the delivery of active agents through the skin using needleless injection systems.

### Background of the Invention

Needleless injectors use compressed gas to accelerate particles to a velocity at which they are capable of penetrating skin and mucosal barriers; such devices are described'in WO-A-94/24263. A requirement is that the particles have mechanical strength, and it is advantageous to have a high density. It is also beneficial to use particles having uniform shape, preferably spherical, and a controlled size distribution; these factors affect the aerodynamic behaviour and the penetration of the particles, and hence the efficacy of the delivery of the active agent. Useful particles typically have a size in the range of 10-500 µm.

The production of solid or dense microparticles can be achieved by milling, e.g. micronisation of larger particles, crystallisation, precipitation or another solution-based microparticle generation technique. However, these techniques typically do not produce spherical microparticles.

A technique which does not normally produce solid microparticles is spray-drying, where often low density particles and agglomerates are formed. A major industry where high density products are important is the dairy industry where skimmed milk powders are produced (Spray Drying Handbook, K. Masters, 5th Edition, 1991, Longman Scientific and Technical, pages 330-336). In this section, products produced by conventional spray-drying are shown on photomicrographs where it is stated that they contain "vacuoles", are of "low density", are thin-walled, "cannot withstand mechanical handling and are readily fragmented", and are obtained together with high and low amounts of occluded air. Some increase in density is described by using a more complicated, two-stage spray-drying process which produces contorted and shrivelled particles. Charlesworth and Marshall, J. Appl. Chem. Eng., 6 No. 1, 9 (1960), describes the morphology of particles produced from spray-drying where all the particles are porous, sponge-like or contain occluded air as a result of collapsing, blistering, bubbling or expansion. Examples of processes in which the inclusion of air is optimised in a spray-drying process are described in WO-A-92/18164, WO-A-96/09814 and WO-A-96/18388.

WO-A-95/34291 discloses pharmaceutical particulate formulations in the form of coated cores containing an inert, inorganic carrier. The formulations may be produced by a process which includes a step of spray drying.

WO-A-96/03979 describes solid forms with controlled release of an active ingredient which can be prepared according to spray drying and spray congealing techniques.

WO-A-97/48485 relates to a method for providing dense particle compositions, for use in transdermal particle delivery, by compaction.

### Summary of the Invention

Surprisingly, it has been found that dense microspheres of solid or semi-solid form can be produced from materials using carefully controlled spray-drying conditions. These microspheres are particularly suitable for use in needleless injection systems due to their density and sphericity. More particularly, the relative particle density may be at least 80% , often at least 90% and even 100% of the solid material. The sphericity is usually such that the shape factor is 1 to 5.

Accordingly, a first aspect of the invention involves a needleless injector as defined in the appended Claim 1.

In a second aspect, the invention provides the use of microparticles as defined in the appended claim 13.

### Description of the Invention

Aspects of the present invention are illustrated, by way of example only, in the accompanying drawings, in which:
Figure 1 shows, schematically, microparticles of the invention;
Figures 2A and 2B are photomicrographs of the product of Example 1;
Figure 3 shows the particle size distribution for the product of Example 1;
Figures 4A and 4B are photomicrographs of the product of Example 2;
Figure 5 shows the particle size distribution for the NT2TRE1 product of Example 3;
Figure 6 is an optical micrograph of the NT2TRE3 product of Example 5 retained after sieving;
Figure 7 shows the size distribution of the sieved products of Example 5;
Figure 8 shows the particle size distribution for the product of Example 7.

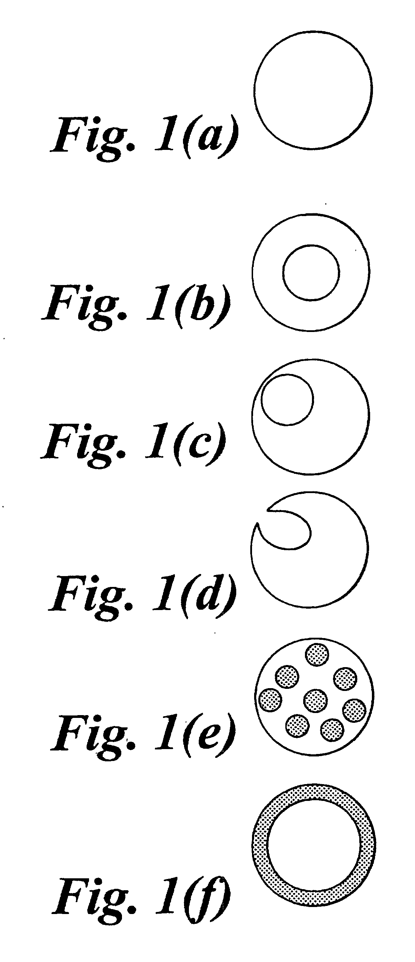

The solid or semi-solid microspheres of the invention produced, also referred to herein as microparticles, can be in a variety of forms, examples of which are shown in Figure 1. In addition to (a) solid spheres, semi-solid spheres can be formed; these are where (b) a small air pocket is occluded in the centre, (c) an occlusion is off centre, or (d) an occlusion has broken out of the microsphere.

Many references, including the Spray Drying Handbook, commonly refer to bulk densities, calculated from the volume which a given mass occupies. In connection with this invention, the particle density is more important; this is based on the volume of the particle including any closed inclusions but not any open structures. Hence, the forms shown in Figure 1(a) and (d) have identical particle densities but (b) and (c) have lower (and identical) particle densities.

A solid microsphere has a particle density identical to the material it is formed from and has a relative particle density of 100%. If small air inclusions are present, the relative particle density is less than 100%. The average particle density can be measured by liquid or gas pycnometry or calculated for individual microspheres using measurements made by optical microscopy. The density of the therapeutic agent is measured at 25°C. From these measurements the microspheres of this invention have relative particle densities of at least 80% and preferably more than 90%, 95%, 99% or 100% of the original material. For application to needleless injection systems, high relative particle densities are required to give mechanical strength and the given relative densities are suitable. In particular, the microspheres can meet the requirements set out, for needleless injection, in WO-A-94/24263.

Active materials, which the microparticles of the invention may comprise or consist of and which may be delivered by needleless injection, are therapeutic agents including pharmacologically active substances, which are generally solids. Therapeutic agents which may be delivered include, for example, proteins, peptides, nucleic acids and small organic molecules, for example local anesthetics (such as cocaine, procaine and lidocaine), hypnotics and sedatives (such as barbiturates, benzodiazepines and chloral derivatives), psychiatric agents (such as phenothiazines, tricyclic antidepressants and monoamine oxidase inhibitors), anti-epilepsy compounds (such as hydantoins), L-dopa, opium-based alkaloids, analgesics, anti-inflammatories, allopurinol, cancer chemotherapeutic agents, anticholinesterases, sympathomimetics (such as epinephrine, salbutamol and ephedrine), antimuscarinics (such as atropine), α-adrenergic blocking agents (such as phentolamine), β-adrenergic blocking agents (such as propranolol), ganglionic stimulating and blocking agents (such as nicotine), neuromuscular blocking agents, autacoids (such as antihistamines and 5-HT antagonists), prostaglandins, plasma kinins (such as bradykinin), cardiovascular drugs (such as digitalis), antiarrhythmic drugs, antihypertensives, vasodilators (such as amyl nitrate and nitroglycerin) diuretics, oxytocin, antibiotics, anthelminthics, fungicides, antiviral compounds (such as acyclovir), anti-trypanosomals, anticoagulants, sex hormones (for example for HRT or contraception), insulin, alprostadil, blood-clotting factors, calcitonin, growth hormones, vaccines, constructs for gene therapy and steroids. The recipient may be a human or any other vertebrate, preferably a mammal, bird or fish for example a cow, sheep, horse, pig, chicken, turkey, dog, cat or salmon, or a plant, especially for DNA transformation of the plant. For example, DNA is generally presented as a plasmid and may, for example, be the DNA encoding an anti-Chlamydia antigen disclosed in Vanrompay *et al* (1999) *Vaccine* 17, 2628-2635. Vaccines may take the form of proteins or other polypeptides or oligopeptides, or DNA encoding an antigen, for example DNA encoding an HIV or hepatitis B antigen. The microspheres may be formed from the active material alone, or they may contain one or more excipients or stabilisers including proteins, sugars, antiseptics, preservatives and buffers. Carbohydrates and other glass-forming substances may be employed as stabilisers or excipients. Preferably, the excipients are parenterally acceptable. If an excipient is present, the active compound may be uniformly distributed or be in the form of smaller particles entrapped in a matrix, as shown in Figure 1(e). Suitable carbohydrates that may be used are as disclosed in WO 96/03978. Hydrophobically derivatised carbohydrates, as disclosed in WO 96/03978, may be used to provide a controlled release form of the particles.

A further embodiment of this invention is the use of excipients or additives with higher density than the active substance or excipient to form even higher density microspheres.

Microspheres of this invention are typically of defined sizes with 95 % or more of the particles (by weight) having a size in the range of 10-500 µm, preferably 20-200 µm, and most preferably 30-100 µm. The modal distribution may be centred around 10 µm bands, i.e. 30, 40, 50, 60, 70, 80, 90 and 100 µm. Preferably, in a monomodal sample, 80% of the particles by weight are within a size range of 10 µm for the particles of a smaller size to a size range of 25 µm for the particles having a larger size (the range increasing with the size of the particles), more preferably, 90% of the particles are within a size range of 15 µm (for the smaller particles) to 30 µm (for the larger particles).

The microspheres of the invention may be formed with a bimodal distribution of particles sizes. Typically, when a rotary atomiser is used, at least 60 % , such as more than 75%, by weight of the particles have particle sizes distributed about one modal size and the remaining particles have particle sizes distributed about a smaller modal size. Where a monomodal particle size distribution is required, the smaller particles may be separated from the larger particles by routine techniques, such as sieving, for example. Microparticles having other distributions of particle sizes can also be obtained in the invention.

The sphericity of the particles is also important and is defined as the shape factor which is the true surface area divided by the equivalent spherical area for the particle volume. The particle surface area can be found by using the standard technique of nitrogen adsorption with subsequent BET analysis. The microspheres of this invention typically have a shape factor of 1 to 5, preferably 1 to 2. Alternative techniques for assessing shape can be found from optical microscopy aided by image analysis to measure circularity and elongation which give similar values to the shape factor.

The microspheres are generally made by spray-drying a solution or suspension of the material. Suitable solvents for most pharmacologically active substances are known. Water is the preferred solvent. The concentration of the material can be varied in order to arrive at the desired solid microparticles but 0.1 to 70% solutions, preferably 10-30% solutions, can be suitable. If the microparticles do not consist of the active material, from the carriers mentioned above, such as a relatively inert protein (such as human serum albumin, preferably produced by rDNA techniques) or sugar (such as trehalose), may be used. Water is again the preferred solvent.

The concentration of active ingredient in the sprayed solution or suspension, and the ratio of the active ingredient to the carrier material (if present) will generally be governed by the amount of the particles to be delivered by the injector and the dose of active ingredient desired.

A conventional spray dryer may be used, e.g. a pilot scale spray dryer atomising the liquid feed solution or suspension by either a pressure nozzle or two fluid atomisation, although rotary atomisers are preferred. The formation of suitable solid or semi-solid microspheres may be dependent on the use of low outlet temperatures in the drying process, for certain therapeutic agents or mixtures of therapeutic agents and excipients. Suitable outlet temperatures can be readily determined by the skilled person for any given therapeutic agent or mixture of therapeutic agent and excipient. The inlet temperature is set to give the required outlet temperature based on the type of atomisation used and other variables such as drying airflow rate; it may be, for example, 50-270°C. The particle size is controlled by standard parameters for the atomiser used at a given feed concentration.

The microspheres may be further dried, following their formation by spray-drying, to remove residual water or solvent by the use of heat and/or vacuum. Suitable drying techniques for this further drying step include, for example, fluidised bed drying. The use of a fluidised bed for this further drying step has the advantage that, when the microspheres have a bimodal particle distribution, the small particles may be separated from the larger particles by elutriation. The formation of crystals should be avoided.

The microspheres may also be coated using standard techniques, e.g. fluid bed coating, to add a further layer or layers to alter the release profile or protect the active compound, as shown in Figure 1 (f). The particle size distribution produced may also be modified to select a particular size range using sieving or other commercial classification techniques to further define particle distribution.

The microspheres may be sterilised, depending on their application. A sterile product can be achieved through either aseptic manufacturing or terminal sterilisation, e.g. gamma irradiation.

Examples of needleless syringes which may be used to deliver the microparticles of the invention and component parts thereof are shown in WO 94/24263 (issued as US 5,899,880 and US 5,630,796).

The syringe is typically some 18 cm long, although it may be smaller or larger than this, and is arranged to be held in the palm of the hand with the thumb overlying the upper end.
In order to carry out an injection, the wider end of the spacer shroud of the device is pressed against a patient's skin. The gas released from a reservoir into a chamber eventually creates in the chamber a pressure sufficient to burst two diaphragms and allow the gas to travel through a nozzle, with the particles entrained thereby, into the patient's skin.

The chamber may be prefilled with gas, such as helium, at a superatmospheric pressure of, say, 2-4 bar, but possibly even as high as 10 bar. The particles of the invention are thus entrained in (ie suspended in) a gas such as helium at the moment of delivery.

The following Examples further illustrate the invention.

### Example 1

100 ml of diafiltered aqueous 20 % w/v (weight by volume) HSA solution (as a model for a pharmacologically active protein, or as the carrier for a pharmacologically active compound) was spray dried on a Niro Mobile Minor spray dryer using a NT2 rotary atomiser (Newland Design, Lancaster) at the following conditions:

| | |
|---|---|
| Inlet Temperature | 245°C |
| Outlet Temperature | 35 °C |
| Feed Rate | 10 g/min |
| Rotational Speed | 30,000 rpm |

The outlet temperature is low as additional air was supplied to guide the droplets into the drying chamber.

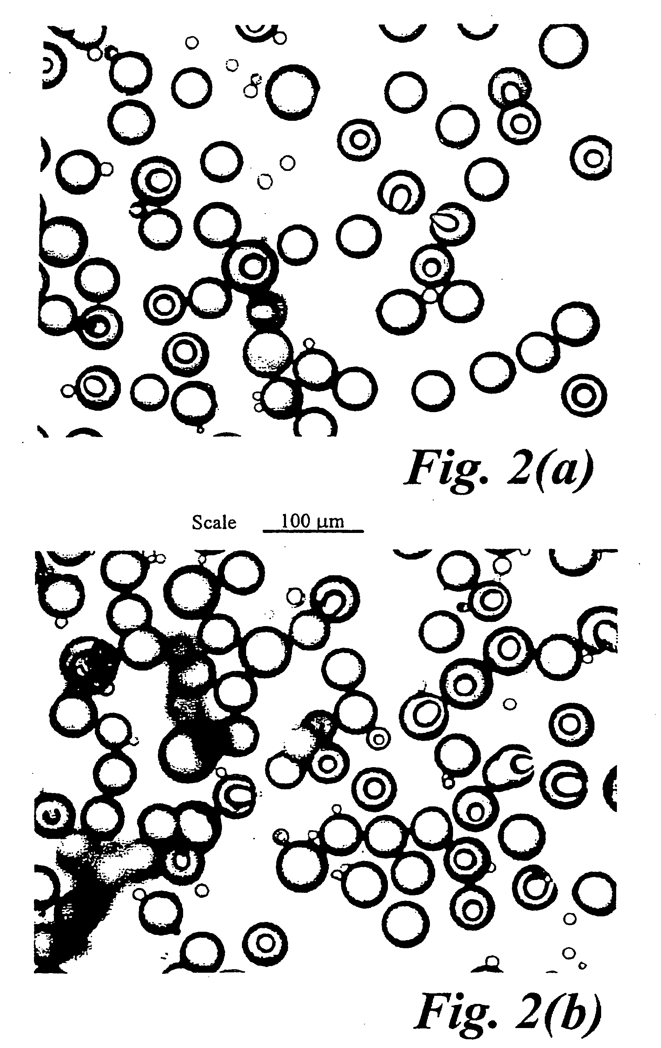

A water soluble product was obtained of which photomicrographs can be found in Figure 2. These show that over 65% of the microspheres were solid with a uniform size of around 50 µm. The similarly sized microspheres containing small amounts of air had thick walls and calculated densities of more than 90 % of the original material forming the microspheres. It is also obvious that the particles are spherical.

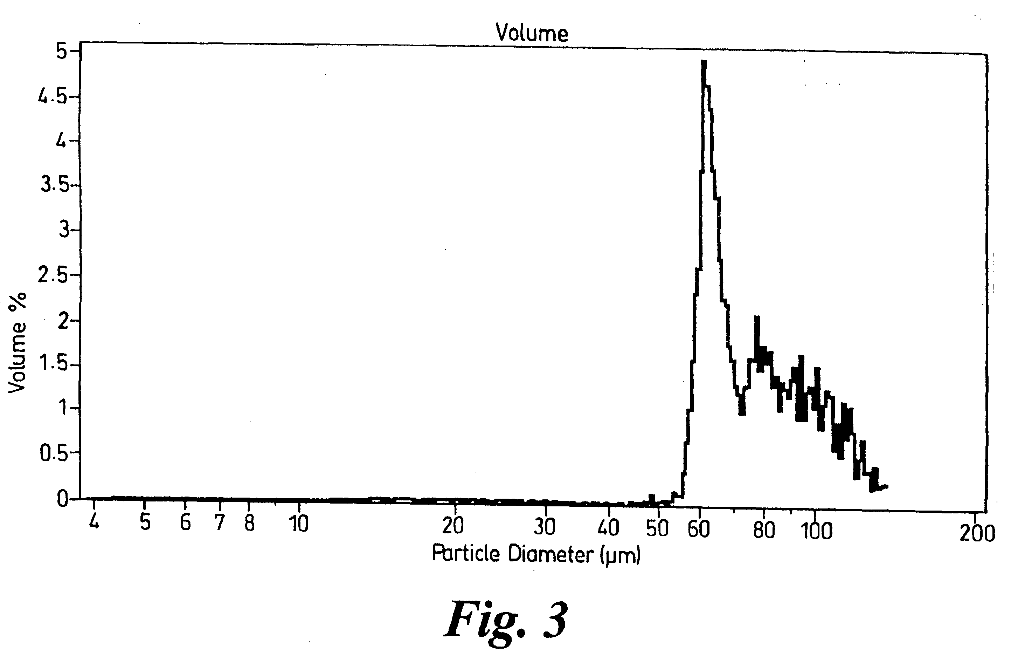

For further size analysis 5 g of the spray dried microcapsules were insolubilised by heating for 55 minutes at a temperature of 176°C in a hot air oven. The microspheres were sized using a Coulter Multisizer 2E (trade mark) and a TAII Sampling Stand fitted with a 200 µm orifice tube which found that the volume median diameter of the microspheres was 71 µm and the modal size was 61 µm This size distribution can be found in Figure 3. The larger size measured by the Coulter Counter is due to swelling of the microsphere in an aqueous environment.

### Example 2

100 ml of diafiltered aqueous 31 % w/v HSA solution (again as a model or carrier) was spray dried on a Niro Mobile Minor spray dryer using the following conditions:

| | |
|---|---|
| Inlet Temperature | 80°C |
| Outlet Temperature | 48°C |
| Atomisation Pressure | 1.0 barg |
| Feed Rate | 13.3 g/min |
| Atomisation Type | Two fluid nozzle |

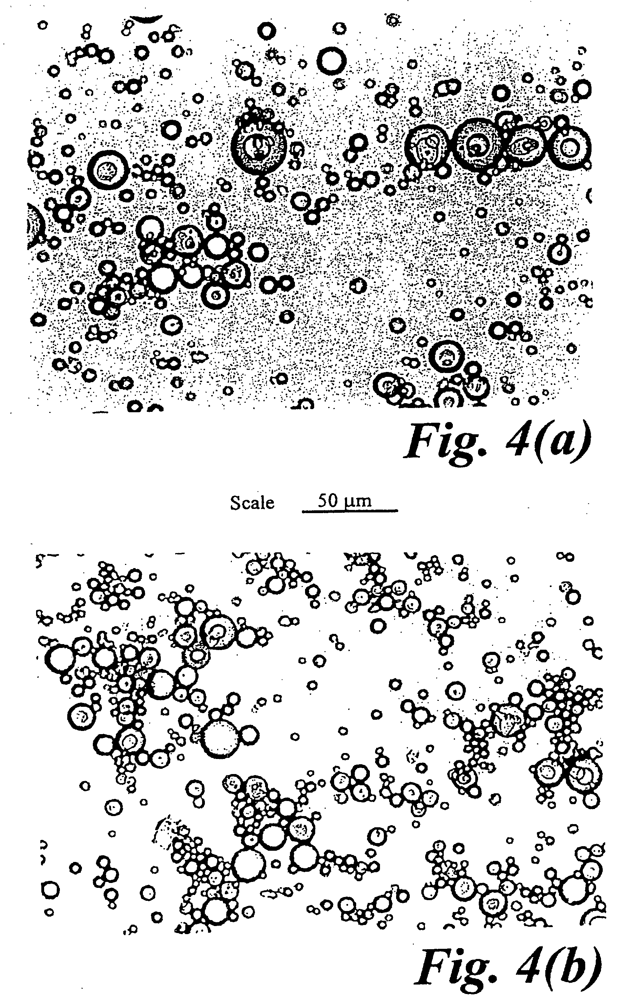

Photomicrographs of the soluble spray dried product can be found in Figure 4. The microspheres are nearly all solid and smaller than the product from Example 1. The minority of microspheres that contain air have thick walls imparting a high mechanical strength.

### Example 3

150 ml of 39% w/v trehalose solution (equivalent to 64g of trehalose dihydrate (Sigma Aldrich Company Ltd, Poole, Dorset) dissolved in water up to a volume of 150 ml) was spray dried on a Niro Mobile Minor spray dryer using a NT2 rotary atomiser (Newland Design, Lancaster) at the following conditions:

| | |
|---|---|
| Inlet Temperature | 200°C |
| Outlet Temperature | 108°C |
| Feed Rate | 6 g/min |
| Rotational Speed | 13,500 rpm |

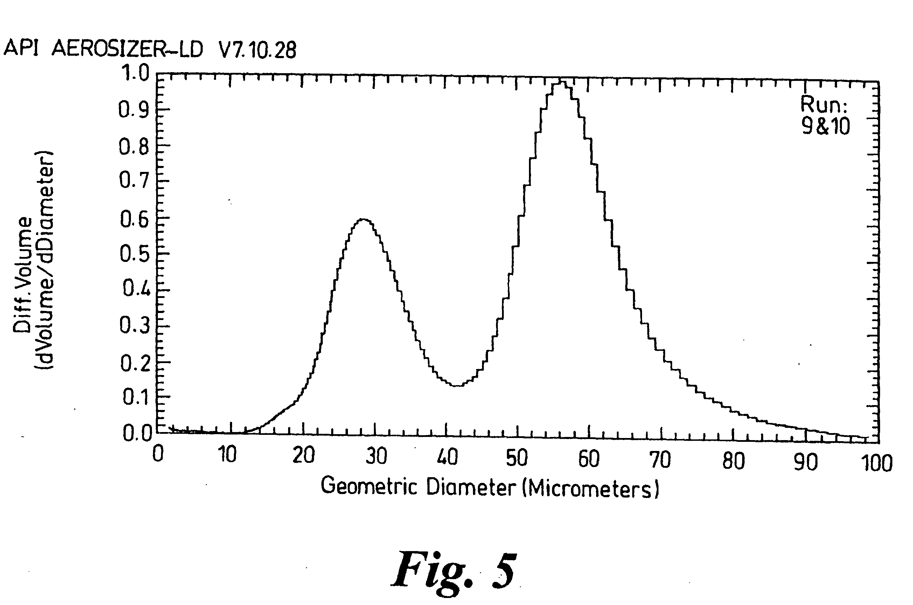

These process conditions gave a product yield of 81%. The product (Batch NT2TRE1) obtained on microscopic examination suspended in vegetable oil showed a bimodal size distribution of microspheres with more than 99% of population solid containing no entrapped air. The geometric size distribution was determined using a API Aerosizer fitted with an Aerodispenser (Amherst Process Instruments Inc, Hadley, MA) using a high shear force, medium feed rate and a particle density of 1.56 g/cm³. The results from this anlysis showed that the main larger peak of the distribution had a modal size of 56 µm with the smaller fraction having a modal size of 28 µm. The size distribution obtained from the Aerosizer is shown in Figure 5.

### Example 4

Example 3 was repeated with the same feed concentration using higher rotational speeds for the NT2 atomiser at 16,400 rpm (Batch NT2TRE2) and 19,000 rpm (Batch NT2TRE3) with similar spray drying conditions. The subsequent microscopic and size analysis using the Aerosizer showed the following results (Table 1). The process yields were 94 and 89% respectively.

**Table 1**

| Batch Number | Atomiser Speed (rpm) | Percentage Solid | Minor Peak Modal Size (µm) | Major Peak Modal Size (µm) |
|---|---|---|---|---|
| NT2TRE2 | 16,400 | > 99 | 22 | 47 |
| NT2TRE3 | 19,000 | > 99 | 19 | 39 |

### Example 5

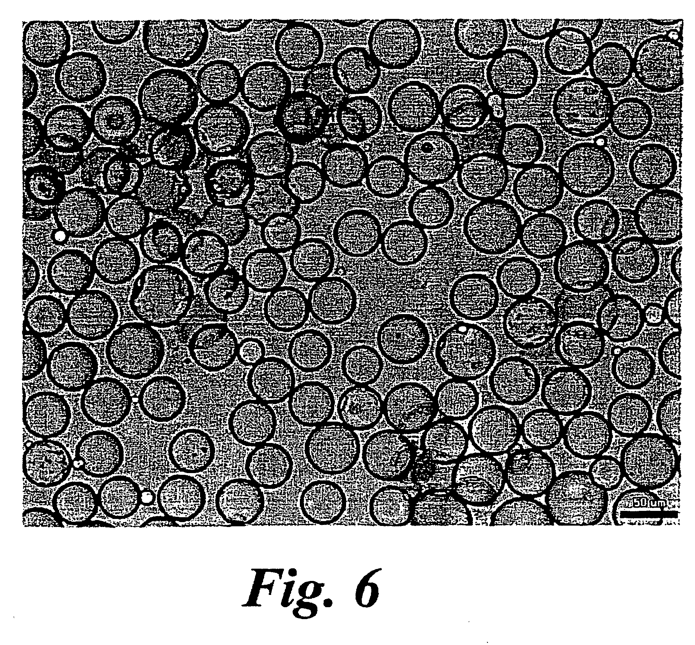

The three products from Examples 3 and 4 were sieved to separate the two peaks of the bimodal distribution. 5g of batch NT2TRE1 was placed in a 200 mm diameter stainless steel test sieve (Endecotts, London) with an aperture size of 38 µm. The sieve was fitted with a lid and receiver and manually shaken for 5 minutes. The materials that were retained by and passed through the sieve were collected for assessment. Similarly 5g of each of the products from batches NT2TRE2 and NT2TRE3 were sieved through 38 and 32 µm sieves respectively. The yield from the larger fraction retained by the sieve was in all cases greater than 60 % . Microscopic examination showed a narrow size distribution and efficient separation of the two peaks of the bimodal size distribution. A photomicrograph of the fraction retained by the 32 µm sieve is shown in Figure 6. The six fractions produced by sieving from the three batches were sized using the Aerosizer to give the results shown in Table 2.

**Table 2**

| Batch Number | Sieve Aperture Size (µm) | Modal Size of Product retained by the Sieve (µm) | Modal Size of Product passed through the Sieve (µm) |
|---|---|---|---|
| NT2TRE1 | 38 | 57 | 28 |
| NT2TRE2 | 38 | 47 | 22 |
| NT2TRE3 | 32 | 40 | 18 |

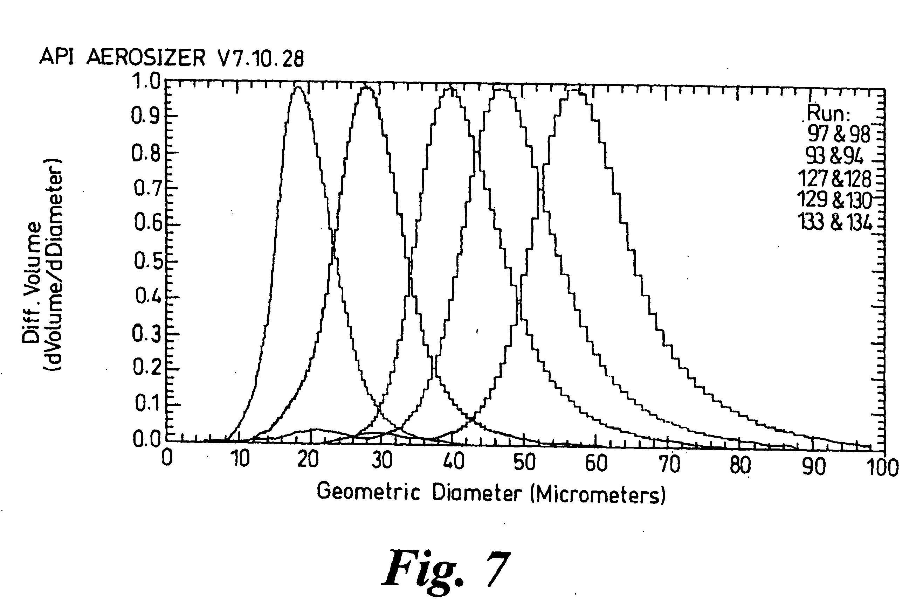

The Aerosizer size distributions are shown in Figure 7 for the microspheres which passed through the sieves for batches NT2TRE3 and NT2TRE1 followed by the microspheres retained by the sieve for batches NT2TRE3, NT2TRE2 and NT2TRE1 in order of increasing size.

On further analysis of the geometric size distributions, the percentage of the particle population was calculated as shown in Table 3.

**Table 3**

| Modal Size (µm) | Lower Size Limit (µm) | Upper Size Limit (µm) | Size Range (µm) | Percentage of Population within Size Range |
|---|---|---|---|---|
| 18 | 16 | 26 | 10 | 70 |
| 28 | 24 | 36 | 12 | 70 |
| 40 | 37 | 53 | 16 | 70 |
| 47 | 43 | 61 | 18 | 70 |
| 57 | 52 | 72 | 20 | 70 |

The product that had a size of 40 µm also showed 75 % of the particles were within a 17 µm size range and similarly 80 % were within a 19µm range.

### Example 6

A feed solution was prepared by dissolving 7g of trehalose octaacetate (Sigma Aldrich Company Ltd, Poole, Dorset) and 3g of nifedipine (Seloc France, Limay) in acetone to a volume of 50 ml. The resulting solution had a total solids loading of 20 % w/v. This feed solution was spray dried on a Niro Mobile Minor spray dryer using the NT2 rotary atomiser using the following conditions:

| | |
|---|---|
| Inlet Temperature | 65°C |
| Outlet Temperature | 46°C |
| Feed Rate | 10 g/min |
| Rotational Speed | 14,600 rpm |

A product yield of 78 % was obtained from these process conditions. The product when assessed using optical microscopy showed a bimodal size distribution of solid microspheres with modal sizes of around 44 µm and 20 µm when compared to a reference graticule.

### Example 7

100 ml of 14% w/v raffinose pentahydrate solution (14g of raffinose pentahydrate (Pfanstiehl, Waukegan, IL) dissolved in water to a volume of 100 ml) was spray dried on a Niro Mobile Minor spray dryer using a NT2 rotary atomiser at the following conditions:

| | |
|---|---|
| Inlet Temperature | 170 ° C |
| Outlet Temperature | 82 ° C |
| Feed Rate | 10g/min |
| Rotational Speed | 13,500 rpm |

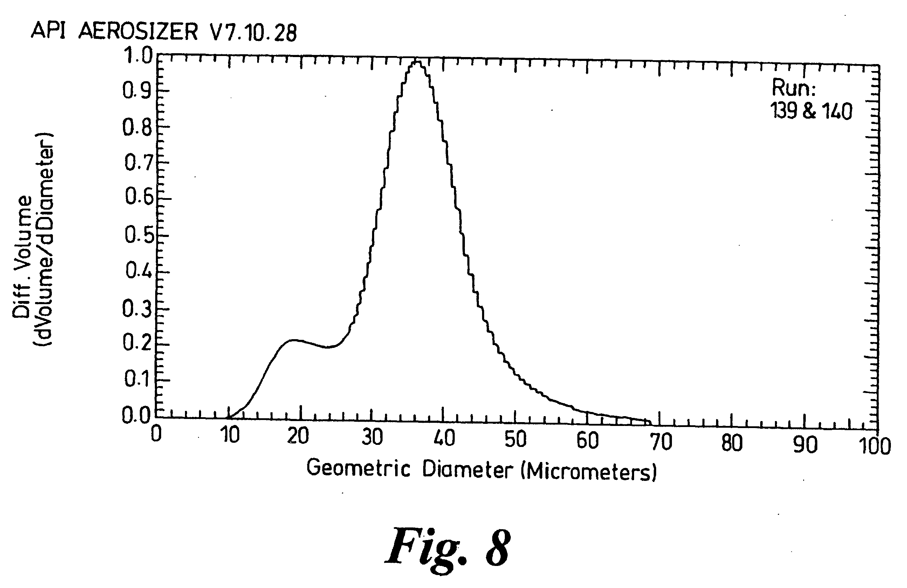

The product obtained, with a process yield of 68 % , showed on microscopic examination a bimodal size distribution of solid microspheres containing no entrapped air. The size distribution was determined on the Aerosizer using the same analytical conditions as Example 3 and a particle density of 1.47 g/cm³. The results from this analysis gave a main larger distribution with modal size of 36 µm with only a very small fraction having a modal size of 18 µm as shown in Figure 8. On analysis of the distribution it was found that 70 % of the microspheres were present within the 17 µm size range between 26 and 43 µm. The raffinose pentahydrate is a carrier for a pharmacologically active compound.

### Example 8

70 ml of a 31 % w/v lidocaine solution in acetone (21.5g of lidocaine (Sigma)) was spray dried on a Niro Mobile Minor spray dryer using a NT2 rotary atomiser at the following conditions:

| | |
|---|---|
| Inlet Temperature | 65°C |
| Outlet Temperature | 45°C |
| Feed Rate | 10 g/min |
| Rotational Speed | 13,500 rpm |

The product was spherical on optical assessment. The particle size distribution was bimodal with spherical solid microspheres having modal sizes of 41 µm and 20 µm.

### Example 9

A solution was prepared by dissolving 38 g of trehalose dihydrate and 2 g diltizem hydrochloride (Lusochimica spa, Milan, Italy) in water to give a total volume of 100 ml. This solution was spray dried using the NT2 atomiser and Mobile Minor spray drier using the following conditions:

| | |
|---|---|
| Inlet Temperature | 200°C |
| Outlet Temperature | 105°C |
| Feed Rate | 11 g/min |
| Rotational Speed | 13,500 rpm |

A process yield of 94 % was obtained. On microscopic examination, the smooth and spherical particles produced exhibited a bimodal size distribution with less than 2 % of the particles containing small amounts of entrapped air. This was confirmed when sized using the Aerosizer, according to the conditions and density described in Example 3. This showed that the major peak which contained the larger microspheres had a modal size of 43 µm and the smaller peak had a mode of 20 µm. The geometric size distribution showed that 70 % of the particle population was in the range of 36 to 56 µm which is a 20 µm size range.

### Example 10

A solution was prepared by dissolving 38 g of trehalose dihydrate and 2 g of a model protein in the form of human serum albumin (Sigma) in water to give a total volume of 100 ml. This solution was spray dried as described in Example 9. In common with Example 9, similar process yields and particle characteristics were obtained. To evaluate whether the spray drying had either degraded or polymerised the albumin, gel electrophoresis under non-reducing conditions was carried out using reference lyophilised albumin and molecular markers. This showed that the albumin was unaffected by the spray drying process. This was also confirmed by gel permeation chromatography which demonstrated that no additional dimerisation or polymerisation had occurred.

## Claims

1. A needleless injector comprising microparticles of a material comprising a therapeutic agent or consisting of a therapeutic agent and, optionally, an excipient, the microparticles having a relative particle density of at least 80 % of the material, and a shape factor, which is defined as the true surface area divided by the equivalent spherical area for the particle volume, of 1 to 5.

2. A needleless injector according to Claim 1, wherein the microparticles are obtainable by spray-drying from solution or suspension.

3. A needleless injector according to Claim 1 or Claim 2, wherein the relative particle density is at least 90%.

4. A needleless injector according to any preceding claim, wherein at least 95 % of the microparticles by weight have a diameter of 10-500 µm.

5. A needleless injector according to Claim 4, wherein at least 95% of the microparticles by weight have a diameter of 20-200 µm.

6. A needleless injector according to Claim 4, wherein at least 95 % of the microparticles by weight have a diameter of 30-100 µm.

7. A needleless injector according to any preceding claim, wherein the shape factor is 1 to 2.

8. A needleless injector according to any preceding claim, wherein the therapeutic agent is a vaccine.

9. A needleless injector according to any preceding claim, wherein the needleless injector uses compressed gas to accelerate particles to a velocity at which they are capable of penetrating skin.

10. A needleless injector according to any preceding claim, wherein the microparticles comprise a carbohydrate or other glass-forming substance.

11. A needleless injector according to any preceding claim, wherein the microparticles further comprise an additive with a higher density than the therapeutic agent and/or the excipient, if present, such that the microparticles have a relative particle density of more than 100% of the material.

12. A needleless injector according to any preceding claim, wherein the microparticles comprise an excipient and the therapeutic agent is uniformly distributed throughout the microparticles or is in the form of smaller particles entrapped in a matrix.

13. Use of microparticles as defined in any preceding claim for the manufacture of a medicament for administration by needleless injection.

## Patentansprüche

1. Nadellose Injektionsvorrichtung, die Mikropartikel eines Materials umfasst, das ein therapeutisches Mittel umfasst oder aus einem therapeutischen Mittel und optional einem Streckmittel besteht, wobei die Mikropartikel eine relative Teilchendichte von mindestens 80 % des Materials und einen Formfaktor, der als die tatsächliche Oberfläche geteilt durch die äquivalente Kugelfläche für das Teilchenvolumen definiert ist, von 1 bis 5 aufweisen.

2. Nadellose Injektionsvorrichtung nach Anspruch 1, wobei die Mikropartikel durch sprühtrocknen aus einer Lösung oder suspension erhältlich sind.

3. Nadellose Injektionsvorrichtung nach Anspruch 1 oder Anspruch 2, wobei die relative Teilchendichte mindestens 90 % beträgt.

4. Nadellose Injektionsvorrichtung nach einem der vorhergehenden Ansprüche, wobei mindestens 95 % der Mikropartikel, bezogen auf das Gewicht, einen Durchmesser von 10-500 µm aufweisen.

5. Nadellose Injektionsvorrichtung nach Anspruch 4, wobei mindestens 95 % der Mikropartikel, bezogen auf das Gewicht, einen Durchmesser von 20-200 µm aufweisen.

6. Nadellose Injektionsvorrichtung nach Anspruch 4, wobei mindestens 95 % der Mikropartikel, bezogen auf das Gewicht, einen Durchmesser von 30-100 µm aufweisen.

7. Nadellose Injektionsvorrichtung nach einem der vorhergehenden Ansprüche, wobei der Formfaktor 1 bis 2 beträgt.

8. Nadellose Injektionsvorrichtung nach einem der vorhergehenden Ansprüche, wobei das therapeutische Mittel ein Impfstoff ist.

9. Nadellose Injektionsvorrichtung nach einem der vorhergehenden Ansprüche, wobei die nadellose Injektionsvorrichtung komprimiertes Gas zur Beschleunigung von Teilchen auf eine Geschwindigkeit, bei der sie Haut durchdringen können, verwendet.

10. Nadellose Injektionsvorrichtung nach einem der vorhergehenden Ansprüche, wobei die Mikropartikel einen Kohlenwasserstoff oder eine andere glasbildende Substanz umfassen.

11. Nadellose Injektionsvorrichtung nach einem der vorhergehenden Ansprüche, wobei die Mikropartikel ferner einen Zusatzstoff mit einer höheren Dichte als das therapeutische Mittel und/oder das Streckmittel, falls dieses vorhanden ist, derart umfassen, dass die Mikropartikel eine relative Teilchendichte von mehr als 100 % des Materials aufweisen.

12. Nadellose Injektionsvorrichtung nach einem der vorhergehenden Ansprüche, wobei die Mikropartikel ein Streckmittel umfassen und das therapeutische Mittel gleichförmig in den Mikropartikeln verteilt ist oder in der Form kleinerer Teilchen, die in einer Matrix eingebettet sind, vorliegt.

13. verwendung von Mikropartikeln gemäß der Definition in einem der vorhergehenden Ansprüche zur Herstellung eines Medikaments zur Verabreichung durch nadellose Injektion.

## Revendications

1. Injecteur sans aiguille comprenant des microparticules d'un matériau comprenant un agent thérapeutique ou consistant en un agent thérapeutique et, facultativement, un excipient, les microparticules ayant une densité relative de particule d'au moins 80% du matériau, et un facteur de forme, qui est défini comme l'aire surfacique vraie divisée par l'aire sphérique équivalente pour le volume d'une particule, de 1 à 5.

2. Injecteur sans aiguille selon la revendication 1, dans lequel les microparticules peuvent être obtenues par séchage par pulvérisation à partir d'une solution ou d'une suspension.

3. Injecteur sans aiguille selon la revendication 1 ou 2, dans lequel la densité relative de particule est d'au moins 90%.

4. Injecteur sans aiguille selon l'une quelconque des revendications précédentes, dans lequel au moins 95% des microparticules en poids ont un diamètre de 10 à 500 µm.

5. Injecteur sans aiguille selon la revendication 4, dans lequel au moins 95% des microparticules en poids ont un diamètre de 20 à 200 µm.

6. Injecteur sans aiguille selon la revendication 4, dans lequel au moins 95% des microparticules en poids ont un diamètre de 30 à 100 µm.

7. Injecteur sans aiguille selon l'une quelconque des revendications précédentes, dans lequel le facteur de forme est de 1 à 2.

8. Injecteur sans aiguille selon l'une quelconque des revendications précédentes, dans lequel l'agent thérapeutique est un vaccin.

9. Injecteur sans aiguille selon l'une quelconque des revendications précédentes, dans lequel l'injecteur sans aiguille utilise du gaz comprimé pour accélérer les particules à une vitesse à laquelle elles sont capables de pénétrer dans la peau.

10. Injecteur sans aiguille selon l'une quelconque des revendications précédentes, dans lequel les microparticules comprennent un glucide ou une autre substance formant un verre.

11. Injecteur sans aiguille selon l'une quelconque des revendications précédentes, dans lequel les microparticules comprennent en outre un additif avec une densité supérieure à l'agent thérapeutique et/ou l'excipient, s'il est présent, de telle sorte que les microparticules ont une densité relative de particule supérieure à 100% du matériau.

12. Injecteur sans aiguille selon l'une quelconque des revendications précédentes, dans lequel les microparticules comprennent un excipient et l'agent thérapeutique est uniformément distribué sur la totalité des microparticules ou se trouve sous la forme de plus petites particules piégées dans une matrice.

13. Utilisation de microparticules telles que définies dans l'une quelconque des revendications précédentes, pour la fabrication d'un médicament pour l'administration par injection sans aiguille.
